# EUROPEAN PATENT APPLICATION

(11) **EP 4 559 901 A1**
(43) Date of publication of application: **28.05.2025**
(21) Application number: 23842937.7
(22) Date of filing: 14.07.2023
(51) Int. Cl.: C07D 249/04, A61K 51/00, C07D 405/06

(54) **RADIOLABELED ACTIVATED ESTER AND PRECURSOR THEREOF**

(30) Priority: 19.07.2022 JP 2022114844
(71) Applicant: Institute of Science Tokyo, Tokyo 152-8550 (JP); National University Corporation Chiba University, Chiba-shi, Chiba 263-8522 (JP); Taiyo Nippon Sanso Corporation, Tokyo 142-8558 (JP)
(72) Inventor: TANAKA, Hiroshi, Tokyo 152-8550 (JP); UEHARA, Tomoya, Chiba-shi, Chiba 260-8675 (JP)
(74) Representative: J A Kemp LLP
(86) International application number: PCT/JP2023/026069
(87) International publication number: WO 2024/019014

(57) **Abstract**

Provided as a precursor of a radioactive halogen-labeled compound is a compound represented by the following general formula (I): 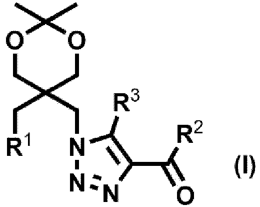 wherein, R¹ represents a leaving group capable of nucleophilic substitution by a halide ion, R² represents a leaving group such that nucleophilic acyl substitution reaction is advanced by an amino group, and R³ represents a hydrogen atom or a halogen atom.

## Description

### [Technical Field]

The present invention relates to a radioactive halogen-labeled compound, its precursor, and a method for manufacturing a radioactive halogen-labeled compound.

### [Background Art]

Radioactive halogen-labeled compounds are widely used in the medical field, including diagnostic methods, such as PET and SPECT, and cancer therapy. In order that a radioactive halogen-labeled compound exhibits diagnostic or therapeutic effect, it is necessary that this compound is stably present in vivo. The present inventors found that since a neopentyl structure has two hydroxy groups and a quaternary carbon with large steric hindrance, a radioactive halogen-labeled compound having this structure can be stably present in vivo (Patent Literatures 1 and 2).

### [Citation List]

### [Patent Literature]

[Patent Literature 1]
   Japanese Patent Laid-Open No. 2017-52713
[Patent Literature 2]
   International Publication No. WO 2019/151384

### [Summary of Invention]

### [Technical Problem]

Radioactive halogens which are used for medical treatment have short half-lives and are therefore required to be obtained from precursors in a short time. Accordingly, the conversion rate from a precursor to a radioactive halogen-labeled compound is important. Against this background, an object of the present invention is to provide a radioactive halogen-labeled compound that is obtained from a precursor with a high conversion rate.

### [Solution to Problem]

The present inventors have intensively studied for solving the above problem and, as a result, found that the conversion rate is significantly improved by introducing a triazole group into a precursor of a radioactive halogen-labeled compound having a neopentyl structure. In addition, it has been found that this triazole group can be easily introduced by a click reaction between an azide and an alkyne.

The present invention has been accomplished based on the above findings.

That is, the present invention provides the following (1) to (12):
(1) A compound represented by the following general formula (I): 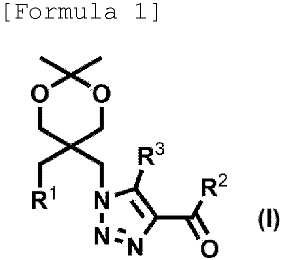 wherein, R¹ represents a leaving group capable of nucleophilic substitution by a halide ion, R² represents a leaving group such that nucleophilic acyl substitution reaction is advanced by an amino group, and R³ represents a hydrogen atom or a halogen atom;
(2) The compound according to (1), wherein R¹ in the general formula (I) is a group represented by the following general formula (A) or (B): 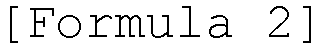 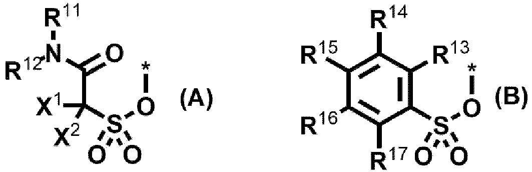 wherein, R¹¹ and R¹² each independently represent an alkyl group having 5 to 20 carbon atoms, X¹ and X² each independently represent a halogen atom, R¹⁵ represents an alkyl group having 4 to 24 carbon atoms or -CONR¹⁸R¹⁹ [wherein, R¹⁸ and R¹⁹ each independently represent an alkyl group having 1 to 24 carbon atoms or an aryl group optionally substituted with a substituent], R¹³, R¹⁴, R¹⁶, R¹⁷, and R¹⁸ each independently represent a hydrogen atom, an alkyl group having 1 to 4 carbon atoms, or an alkoxy group having 1 to 4 carbon atoms, and * represents a binding site;
(3) The compound according to (1), wherein R² in the general formula (I) is a group represented by the following general formula (C), (D), (E), or (F): 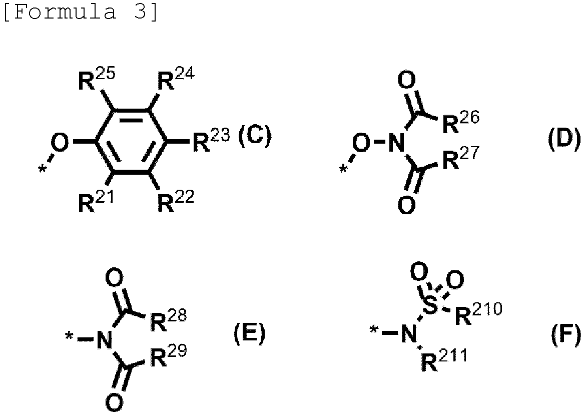 wherein, R²¹, R²², R²³, R²⁴, and R²⁵ each independently represent a hydrogen atom or a halogen atom, R²⁶ and R²⁷ each independently represent a hydrogen atom, an alkyl group having 1 to 4 carbon atoms, or an aryl group optionally substituted with a substituent [R²⁶ and R²⁷ optionally bind to each other to form a ring], R²⁸ and R²⁹ each independently represent a hydrogen atom, an alkyl group having 1 to 4 carbon atoms, an alkoxy group having 1 to 4 carbon atoms, an amino group [one or two hydrogen atoms of the amino group are optionally replaced with an alkyl group having 1 to 4 carbon atoms], or an aryl group optionally substituted with a substituent [R²⁸ and R²⁹ optionally bind to each other to form a ring], R²¹⁰ and R²¹¹ each independently represent a hydrogen atom, an alkyl group having 1 to 4 carbon atoms, or an aryl group optionally substituted with a substituent [R²¹⁰ and R²¹¹ optionally bind to each other to form a ring], and * represents a binding site;
(4) A compound represented by the following general formula (IIa) or (IIb): 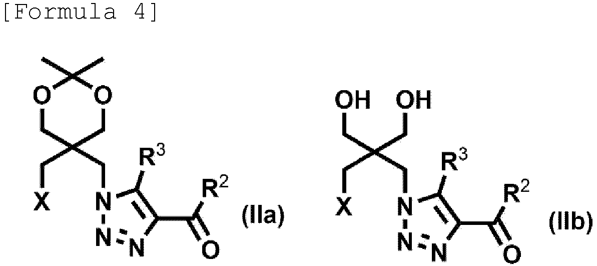 wherein, X represents a radioactive halogen atom, R² represents a leaving group such that nucleophilic acyl substitution reaction is advanced by an amino group, and R³ represents a hydrogen atom or a halogen atom;
(5) The compound according to (4), wherein X in the general formula (IIa) or (IIb) is a radioactive bromine atom, a radioactive iodine atom, or a radioactive astatine atom;
(6) The compound according to (4), wherein R² in the general formula (IIa) or (IIb) is a group represented by the following general formula (C), (D), (E), or (F): 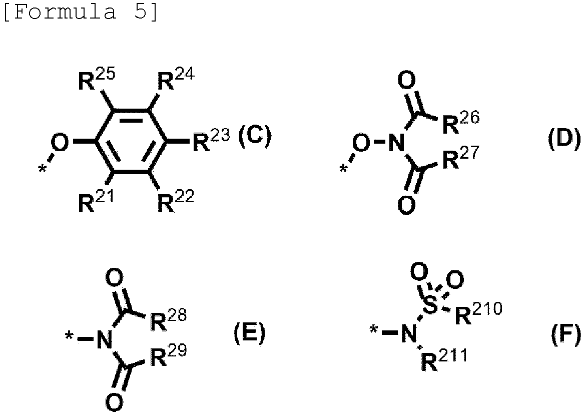 wherein, R²¹, R²², R²³, R²⁴, and R²⁵ each independently represent a hydrogen atom or a halogen atom, R²⁶ and R²⁷ each independently represent a hydrogen atom, an alkyl group having 1 to 4 carbon atoms, or an aryl group optionally substituted with a substituent [R²⁶ and R²⁷ optionally bind to each other to form a ring], R²⁸ and R²⁹ each independently represent a hydrogen atom, an alkyl group having 1 to 4 carbon atoms, an alkoxy group having 1 to 4 carbon atoms, an amino group [one or two hydrogen atoms of the amino group are optionally replaced with an alkyl group having 1 to 4 carbon atoms], or an aryl group optionally substituted with a substituent [R²⁸ and R²⁹ optionally bind to each other to form a ring], R²¹⁰ and R²¹¹ each independently represent a hydrogen atom, an alkyl group having 1 to 4 carbon atoms, or an aryl group optionally substituted with a substituent [R²¹⁰ and R²¹¹ optionally bind to each other to form a ring], and * represents a binding site;
(7) A compound represented by the following general formula (IIIa) or (IIIb): 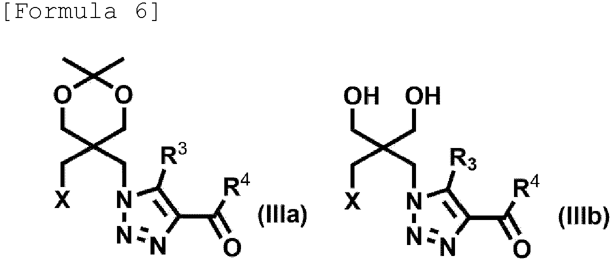 wherein, X represents a radioactive halogen atom, R³ represents a hydrogen atom or a halogen atom, and R⁴ represents a monovalent group derived from a physiologically active substance;
(8) The compound according to (7), wherein X in the general formula (IIIa) or (IIIb) is a radioactive bromine atom, a radioactive iodine atom, or a radioactive astatine atom;
(9) The compound according to (7), wherein R⁴ in the general formula (IIIa) or (IIIb) is a monovalent group derived from a physiologically active substance that accumulates at a disease site;
(10) The compound according to (7), wherein R⁴ in the general formula (IIIa) or (IIIb) is a monovalent group derived from an antibody;
(11) A method for manufacturing the compound according to any one of (4) to (6), comprising a step of reacting the compound according to any one of (1) to (3) with a radioactive halide ion; and
(12) A method for manufacturing the compound according to any one of (7) to (10), comprising a step of reacting the compound according to any one of (4) to (6) with a physiologically active substance.

The present specification encompasses the contents described in the description and/or drawings of Japanese Patent Application No. 2022-114844, which is a priority document of the present application.

### [Advantageous Effects of Invention]

The present invention provides a new radioactive halogen-labeled compound. This compound can be obtained from a precursor with a high conversion rate, and the precursor can be easily synthesized utilizing a click reaction.

### [Brief Description of Drawings]

[Figure 1] Figure 1 shows HPLC chromatograms of compound (3).
[Figure 2] Figure 2 shows HPLC chromatograms of compound (4).

### [Description of Embodiments]

The present invention will now be described in detail.

In the present invention, the "halogen atom" is, for example, a fluorine atom, a chlorine atom, a bromine atom, an iodine atom, or an astatine atom.

In the present invention, the "radioactive halogen atom" is, for example, ¹⁸F, ⁷⁵Br, ⁷⁶Br, ⁷⁷Br, ⁸²Br, ¹²³I, ¹²⁴I, ¹²⁵I, ¹³¹I, ¹³³I, ²⁰⁹At, ²¹⁰At, or ²¹¹At.

In the present invention, the "radioactive halide ion" is, for example, ¹⁸F⁻, ⁷⁵Br⁻, ⁷⁶Br⁻, ⁷⁷Br⁻, ⁸²Br⁻, ¹²³I⁻, ¹²⁴I⁻, ¹²⁵I⁻, ¹³¹I⁻, ¹³³I⁻, ²⁰⁹At⁻, ²¹⁰At⁻, or ²¹¹At⁻.

In the present invention, the "alkyl group having 5 to 20 carbon atoms" is a linear or branched alkyl group having 5 or more and 20 or less carbon atoms, and examples thereof include a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an icosyl group.

In the present invention, the "alkyl group having 7 to 11 carbon atoms" is a linear or branched alkyl group having 7 or more and 11 or less carbon atoms, and examples thereof include a heptyl group, an octyl group, a nonyl group, a decyl group, and an undecyl group.

In the present invention, the "alkyl group having 4 to 24 carbon atoms" is a linear or branched alkyl group having 4 or more and 24 or less carbon atoms, and examples thereof include a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, and a tetracosyl group.

In the present invention, the "alkyl group having 1 to 24 carbon atoms" is a linear or branched alkyl group having 1 or more and 24 or less carbon atoms, and examples thereof include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, and a tetracosyl group.

In the present invention, the "alkyl group having 1 to 4 carbon atoms" is a linear or branched alkyl group having 1 or more and 4 or less carbon atoms, and examples thereof include a methyl group, an ethyl group, a propyl group, an iso-propyl group, a butyl group, an iso-butyl group, a sec-butyl group, and a tert-butyl group.

In the present invention, the "alkoxy group having 1 to 4 carbon atoms" is a linear or branched alkoxy group having 1 or more and 4 or less carbon atoms, and examples thereof include a methoxy group, an ethoxy group, a propoxy group, an iso-propoxy group, a butoxy group, an iso-butoxy group, a sec-butoxy group, and a tert-butoxy group.

In the present invention, the "aryl group" is, for example, a phenyl group, a naphthalen-1-yl group, or a naphthalen-2-yl group.

In the present invention, the "aryloxy group" is, for example, a phenoxy group, a naphthalen-1-yloxy group, or a naphthalen-2-yloxy group.

In the general formula (I), R¹ represents a leaving group capable of nucleophilic substitution by a halide ion. Such a leaving group is often used in manufacturing of radioactive halogen-labeled compounds (for example, leaving groups described in Japanese Patent Laid-Open No. 2017-52713, International Publication No. WO 2018/164043, and International Publication No. WO 2019/151384) and is well known to those skilled in the art. Examples of the leaving group capable of nucleophilic substitution by a halide ion include groups represented by the following general formula (A) or (B):

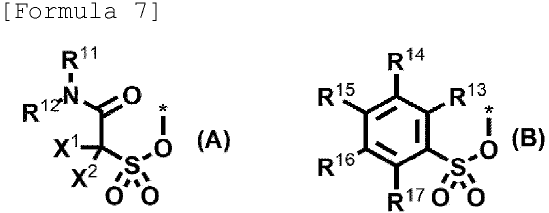

In the general formula (A), R¹¹ and R¹² each independently represent an alkyl group having 5 to 20 carbon atoms. R¹¹ and R¹² may be groups different from each other, but are preferably the same group. R¹¹ and R¹² may be any of the above-mentioned groups and are preferably alkyl groups having 7 to 11 carbon atoms and more preferably alkyl groups having 7 or 11 carbon atoms.

In the general formula (A), X¹ and X² each independently represent a halogen atom. X¹ and X² may be atoms different from each other, but are preferably the same atom. X¹ and X² may be any of halogen atoms and are preferably fluorine atoms or chlorine atoms and more preferably fluorine atoms.

In the general formula (B), R¹⁵ represents an alkyl group having 4 to 24 carbon atoms or -CONR¹⁸R¹⁹. The "alkyl group having 4 to 24 carbon atoms" is preferably a linear alkyl group and more preferably a linear alkyl group having 8 to 16 carbon atoms. In "-CONR¹⁸R¹⁹", R¹⁸ and R¹⁹ each independently represent an alkyl group having 1 to 24 carbon atoms or an aryl group optionally substituted with a substituent. The "alkyl group having 1 to 24 carbon atoms" is preferably a linear alkyl group and more preferably a linear alkyl group having 8 to 16 carbon atoms. The "aryl group" in the "aryl group optionally substituted with a substituent" may be a monocyclic aryl group such as a phenyl group, but is preferably a condensed polycyclic aryl group such as a naphthyl group. Examples of the "substituent" in the "aryl group optionally substituted with a substituent" include an alkyl group having 1 to 4 carbon atoms, an alkoxy group having 1 to 4 carbon atoms, and a halogen atom. R¹⁸ and R¹⁹ may be groups different from each other, but are preferably the same group.

In the general formula (B), R¹³, R¹⁴, R¹⁶, R¹⁷, and R¹⁸ each independently represent a hydrogen atom, an alkyl group having 1 to 4 carbon atoms, or an alkoxy group having 1 to 4 carbon atoms. R¹³, R¹⁴, R¹⁶, R¹⁷, and R¹⁸ may all be the same group, a part of them may be the same group, or all of them may be different groups. R¹³, R¹⁴, R¹⁶, R¹⁷, and R¹⁸ may be any of the above-mentioned groups and are preferably hydrogen atoms.

In the general formulae (I), (IIa), and (IIb), R² represents a leaving group such that nucleophilic acyl substitution reaction is advanced by an amino group. Examples of such a leaving group include groups represented by the following general formula (C), (D), (E), or (F):

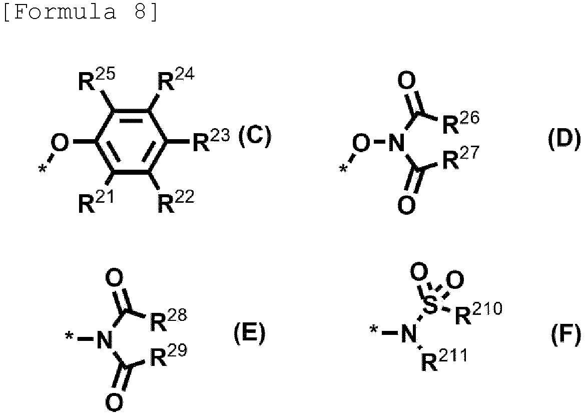

In the general formula (C), R²¹, R²², R²³, R²⁴, and R²⁵ each independently represent a hydrogen atom or a halogen atom. R²¹, R²², R²³, R²⁴, and R²⁵ may be different atoms or the same atom. R²³ may be any of the above-mentioned atoms and is preferably a hydrogen atom or a fluorine atom. R²¹, R²², R²⁴, and R²⁵ may be any of the above-mentioned atoms and are preferably fluorine atoms.

In the general formula (D), R²⁶ and R²⁷ each independently represent a hydrogen atom, an alkyl group having 1 to 4 carbon atoms, or an aryl group optionally substituted with a substituent. Examples of the "substituent" in the "aryl group optionally substituted with a substituent" include an alkyl group having 1 to 4 carbon atoms, an alkoxy group having 1 to 4 carbon atoms, and a halogen atom. R²⁶ and R²⁷ may be groups different from each other or may be the same group. R²⁶ and R²⁷ may bind to each other to form a ring. The ring may be a 5-membered ring, a 6-membered ring, a 7-membered ring, or an 8-membered ring.

In the general formula (E), R²⁸ and R²⁹ each independently represent a hydrogen atom, an alkyl group having 1 to 4 carbon atoms, an alkoxy group having 1 to 4 carbon atoms, an amino group [one or two hydrogen atoms of the amino group are optionally replaced with an alkyl group having 1 to 4 carbon atoms], or an aryl group optionally substituted with a substituent. Examples of the "substituent" in the "aryl group optionally substituted with a substituent" include an alkyl group having 1 to 4 carbon atoms, an alkoxy group having 1 to 4 carbon atoms, and a halogen atom. R²⁸ and R²⁹ may be groups different from each other or may be the same group. R²⁸ and R²⁹ may bind to each other to form a ring. The ring may be a 5-membered ring, a 6-membered ring, a 7-membered ring, or an 8-membered ring.

In the general formula (F), R²¹⁰ and R²¹¹ each independently represent a hydrogen atom, an alkyl group having 1 to 4 carbon atoms, or an aryl group optionally substituted with a substituent. Examples of the "substituent" in the "aryl group optionally substituted with a substituent" include an alkyl group having 1 to 4 carbon atoms, an alkoxy group having 1 to 4 carbon atoms, and a halogen atom. R²¹⁰ and R²¹¹ may be groups different from each other or may be the same group. R²¹⁰ and R²¹¹ may bind to each other to form a ring. The ring may be a 4-membered ring, a 5-membered ring, a 6-membered ring, a 7-membered ring, or an 8-membered ring.

In the general formulae (I), (IIa), (IIb), (IIIa), and (IIIb), R³ represents a hydrogen atom or a halogen atom. R³ may be a hydrogen atom or a halogen atom and is preferably a hydrogen atom.

In the general formulae (IIa), (IIb), (IIIa), and (IIIb), X represents a radioactive halogen atom. The type of the halogen atom is not particularly limited, but is preferably a bromine atom, an iodine atom, or an astatine atom. X may be any radioactive halogen atom and is preferably ¹⁸F, ¹²³I, ¹³¹I, or ²¹¹At and more preferably ¹²³I, ¹³¹I, or ²¹¹At.

In the general formulae (IIIa) and (IIIb), R⁴ represents a monovalent group derived from a physiologically active substance. Here, the term "physiologically active substance" means a substance involved in maintenance or regulation of a physiological function, and examples thereof include an amino acid, a peptide, a protein (including an antibody), and a saccharide. The term "monovalent group derived from a physiologically active substance" means a monovalent group obtained by, for example, removing one hydrogen atom in a molecule of the physiologically active substance. The hydrogen atom to be removed is, for example, when the physiologically active substance includes a hydroxy group or an amino group, a hydrogen atom included in these groups. Since the compound represented by the general formula (IIIa) or (IIIb) is used for diagnosis or therapy of a disease, in order to deliver this compound to a disease site, the physiologically active substance preferably has a property of accumulating at the disease site. Examples of the disease include cancer and Alzheimer disease. When the disease is cancer, the physiologically active substance can be a substance that specifically binds to a cancer cell, and when the disease is Alzheimer disease, the physiologically active substance can be a substance that has affinity to amyloid β or tau protein.

The compound represented by the general formula (I) can be used as a labeled precursor reagent of the radioactive halogen-labeled compound. The labeled precursor reagent of the radioactive halogen-labeled compound is usually composed of only a compound represented by the general formula (I), but may include another substance. The compound represented by the general formula (I) can be manufactured by reacting a compound including an alkyne in the molecule with an azide compound. More specifically, the compound can be manufactured according to the manufacturing method described in Example 1 modified or amended as necessary.

The compound represented by the general formula (I) has advantages as a precursor compound of the radioactive halogen-labeled compound as follows:
1) Since the compound has a halogenation conversion rate higher than those of known precursor compounds, a radioactive halogen-labeled compound can be manufactured in a short time; and
2) Since the compound can be synthesized by a click reaction between a compound including an alkyne in the molecule and an azide compound, the structures of both compounds are not destroyed during the synthesis.

The compound represented by the general formula (IIa) or (IIb) can be used as the radioactive halogen-labeled reagent. The radioactive halogen-labeled reagent is usually composed of only a compound represented by the general formula (IIa) or (IIb), but may include another substance. The compound represented by the general formula (IIa) or (IIb) can be manufactured by reacting a compound represented by the general formula (I) with a radioactive halide ion. The radioactive halide ion to be used is not particularly limited and is preferably ¹⁸F, ¹²³I, ¹³¹I, or ²¹¹At and more preferably ¹²³I, ¹³¹I, or ²¹¹At. The reaction conditions can be appropriately set depending on the compound represented by the general formula (I) and the type of the radioactive halide ion. Those skilled in the art can easily determine the appropriate reaction temperature, reaction time, concentration of each substance, and so on.

The compound represented by the general formula (IIIa) or (IIIb) can be used as a therapeutic agent or diagnostic agent of a disease. The therapeutic or diagnostic agent for a disease may include a substance other than the compound represented by the general formula (IIIa) or (IIIb).

The compound represented by the general formula (IIIa) or (IIIb) can be manufactured by reacting a compound represented by the general formula (IIa) or (IIb) with a physiologically active substance. The physiologically active substance is not particularly limited and is preferably a physiologically active substance having a property of accumulating at a disease site. The reaction conditions can be appropriately set depending on the compound represented by the general formula (IIa) or (IIb) and the type of the physiologically active substance. Those skilled in the art can easily determine the appropriate reaction temperature, reaction time, concentration of each substance, and so on.

### [Examples]

The present invention will now be described in further detail by Examples, but is not limited to these Examples.

### [Example 1] Synthesis of non-radioactive halogen-labeled compound

### General information

NMR spectra were recorded using JEOL Model ECP-400 (400 MHz for ¹H) and Bruker AVANCE III HD 400 (400 MHz for ¹H) in assigned solvents. Chemical shifts were reported in a unit of parts per million (ppm) relative to the signal of internal tetramethylsilane (¹H: 0 ppm) in a CDCl₃ solution. ¹H NMR spectral data were reported as follows. CDCl₃ (7.26 ppm) and CD₃OD-d₄ (3.31 ppm) were used as external standards, and multiplicities were reported using the following abbreviations: s, singlet; br-s, broaded-singlet; d, doublet; br-d, broaded-doublet; dd, doublet of doublets; br-dd, broaded-doublet of doublets; t, triplet; dq, doublet of quartets; q, quartet; m, multiplet; and J, coupling constants in Hertz.

All reactions were monitored by thin-layer chromatography using a 0.2 mm E. Merck silica gel plate (60F-254) and visualized with a p-anisaldehyde solution, ceric sulfate, or ethanol type phosphomolybdic acid and irradiating with UV light. Separation by column chromatography was performed using silica gel (Merck silica gel 60, 0.063 to 0.200 mm).

### Synthesis of (5-(azidomethyl)-2,2-dimethyl-1,3-dioxan-5-yl)methanol (6)

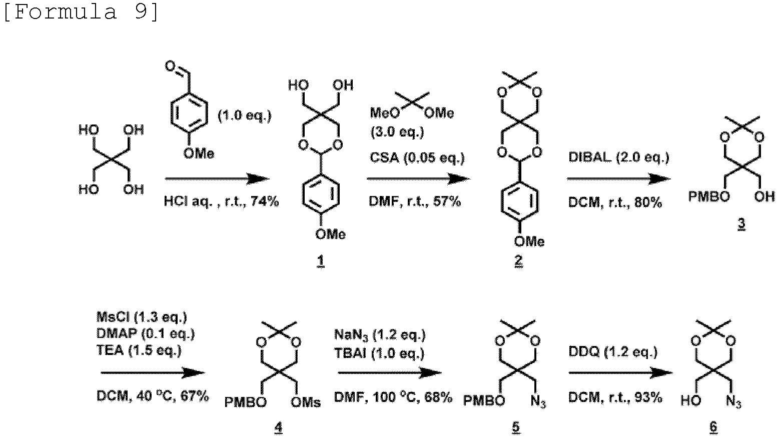

### 9-(4-Methoxyphenyl)-3,3-dimethyl-2,4,8,10-tetraoxaspiro[5.5]undecane (2)

2,2-Dimethoxypropane (1.4 mL, 11.8 mmol, 3.0 eq.) was added to a stirring solution of (2-(4-methoxyphenyl)-1,3-dioxane-5,5-diyl)dimethanol (1) (1.00 g, 3.93 mmol, 1.0 eq.) and (+)-10-camphorsulfonic acid (45.7 mg, 197 µmol, 0.05 eq.) in dry DMF (8 mL) at room temperature in an Ar atmosphere. The mixture was stirred at room temperature for 2 hours, and the reaction mixture was then poured in NaHCO₃ aq. and EtOAc. The aqueous layer was extracted with EtOAc twice. The extracts were combined and were washed with brine, dried over MgSO₄, filtered, and concentrated under reduced pressure. The residue was recrystallized from hexane/EtOAc for purification to obtain 9-(4-methoxyphenyl)-3,3-dimethyl-2,4,8,10-tetraoxaspiro[5.5]undecane (2) (659 mg, 2.24 mmol, 57%) as a white crystal. 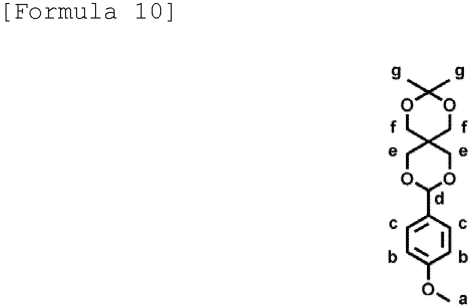 ¹H NMR (400 MHz, CDCl₃) δ 7.33 (d, 2H, H-c, J = 8.8 Hz), 3.23 (s, 2H, H-g), 6.90 (d, 2H, H-c, J = 8.8 Hz), 5.34 (s, 1H, H-d,), 4.58 (d, 1H, H-h), 3.82 (m, 4H, H-e), 3.75 (s, 3H, H-a), 3.66 (s, 2H, H-f)

### (5-(((4-methoxybenzyl)oxy)methyl)-2,2-dimethyl-1,3-dioxan-5-yl)methanol (3)

Diisobutylaluminum hydride (19% in hexane, about 1.0 mol/L) (20.4 mL, 20.4 mmol, 2.0 eq.) was added to a stirring solution of 9-(4-methoxyphenyl)-3,3-dimethyl-2,4,8,10-tetraoxaspiro[5.5]undecane (2) (3.00 g, 10.2 mmol, 1.0 eq.) in dichloromethane (20 mL) at 0°C in an Ar atmosphere. The mixture was stirred at room temperature for 3 hours, and the reaction mixture was then quenched with MeOH, potassium sodium L-(+)-tartrate tetrahydrate, and EtOAc. The aqueous layer was extracted with EtOAc twice. The extracts were combined and were washed with brine, dried over MgSO₄, filtered, and concentrated under reduced pressure. The residue was purified by chromatography using hexane/EtOAc (4 : 1) in the silica gel column to obtain (5-(((4-methoxybenzyl)oxy)methyl)-2,2-dimethyl-1,3-dioxan-5-yl)methanol (3) (2.42 g, 8.17 mmol, 80%) in a colorless oil form. 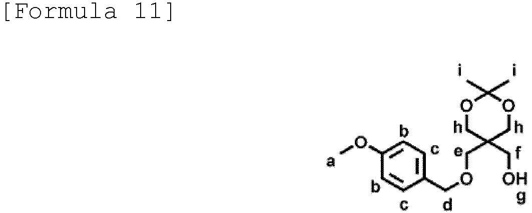 ¹H NMR (400 MHz, CDCl₃) δ 7.24 (d, 2H, H-, J = Hz), 6.88 (d, 2H, H-, J = Hz), 4.46 (s, 2H, H-d), 3.81 (s, 3H, H-a), 3.67 (d, 2H, H-f, J = 6.0 Hz), 3.55 (s, 2H, H-e), 2.37 (t, 1H, H-g, J = 6.0 Hz), 1.40 (d, 6H, H-I, J = 5.1 Hz)

### (5-(((4-Methoxybenzyl)oxy)methyl)-2,2-dimethyl-1,3-dioxan-5-yl)methyl methanesulfonate (4)

Methanesulfonyl chloride (1.00 mL, 12.9 mmol, 1.48 g/mL, 1.3 eq.) was added to a stirring solution of (5-(((4-methoxybenzyl)oxy)methyl)-2,2-dimethyl-1,3-dioxan-5-yl)methanol (3) (2.95 g, 9.95 mmol, 1.0 eq.), triethylamine (2.07 mL, 14.9 mmol, 0.730 g/mL, 1.5 eq.), and N,N-dimethylpyridine-4-amine (122 mg, 995 µmol, 0.1 eq.) in dichloromethane (20 mL) at 0°C in an Ar atmosphere. The mixture was stirred at 40°C for 24 hours, and the reaction mixture was then poured in NH₄Cl aq. and EtOAc. The aqueous layer was extracted with EtOAc twice. The extracts were combined and were washed with brine, dried over MgSO₄, filtered, and concentrated under reduced pressure. The residue was purified by chromatography using hexane/EtOAc (9 : 1) in the silica gel column to obtain (5-(((4-methoxybenzyl)oxy)methyl)-2,2-dimethyl-1,3-dioxan-5-yl)methyl methanesulfonate (4) (2.50 g, 6.68 mmol, 67%) as a colorless oil. 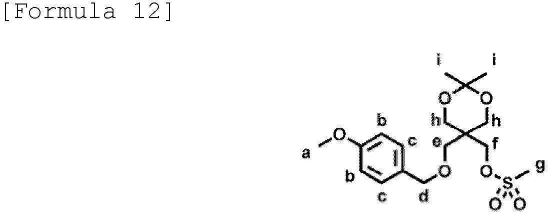 ¹H NMR (400 MHz, CDCl₃) δ 7.22 (d, 2H, J = 8.4 Hz), 6.88 (d, 2H, J = 8.4 Hz), 4.42 (s, 2H, H-d), 4.35 (s, 2H, H-f), 3.80 (s, 2H, H-e), 3.93, 3.69 (d, 2H, H-h, J = 14.8 Hz), 3.35 (s, 2H, H-e), 2.97 (s, 3H, H-g), 1.34 (d, 6H, H-I, J = 4.2 Hz)

### 5-(Azidomethyl)-5-((4-methoxybenzyl)oxy)methyl)-2,2-dimethyl-1,3-dioxane (5)

Sodium azide (521 mg, 8.01 mmol, 1.2 eq.) and tetrabutylammonium iodide (2.47 g, 6.68 mmol, 1.0 eq.) were added to a stirring solution of 5-(((4-methoxybenzyl)oxy)methyl)-2,2-dimethyl-1,3-dioxan-5-yl)methyl methanesulfonate (4) (2.50 g, 6.68 mmol, eq.) in dry DMF (10 mL) at room temperature in an Ar atmosphere. The mixture was stirred at 100°C for 5 hours, and the reaction mixture was then poured in water and EtOAc. The aqueous layer was extracted with EtOAc twice. The extracts were combined and were washed with brine, dried over MgSO₄, filtered, and concentrated under reduced pressure. The residue was purified by chromatography using hexane/EtOAc (9 : 1) in the silica gel column to obtain 5-(azidomethyl)-5-(((4-methoxybenzyl)oxy)methyl)-2,2-dimethyl-1,3-dioxane (5) (1.45 g, 4.51 mmol, 68%) in a colorless oil form. 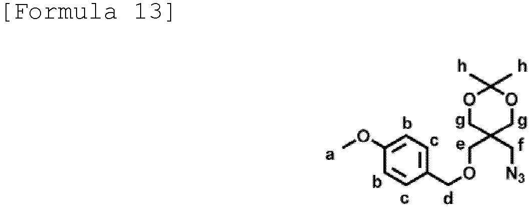 ¹H NMR (400 MHz, CDCl₃) δ 7.23 (d, 2H, H-c, J = 8.6 Hz), 6.89 (d, 2H, H-b, J = 8.6 Hz), 4.32 (s, 2H, H-d), 3.81, (s, 3H, H-a), 3.73, 3.64 (d, 2H, H-g, J = 12.0 Hz), 3.52 (s, 2H, H-f), 3.34 (s, 2H, H-e), 1.39 (s, 6H, H-h)

### (5-(Azidomethyl)-2,2-dimethyl-1,3-dioxan-5-yl)methanol (6)

2,3-dichloro-5,6-dicyano-1,4-benzoquinone (409 mg, 1.80 mmol, 1.2 eq.) was added to a solution of 5-(azidomethyl)-5-(((4-methoxybenzyl)oxy)methyl)-2,2-dimethyl-1,3-dioxane (5) (482 mg, 1.50 mmol, 1.0 eq.) in DCM (2.7 mL) and a phosphate buffer solution pH 7.4 (300 µL). The mixture was stirred at room temperature for 45 minutes, and the reaction mixture was then poured in NaHCO₃ aq. and EtOAc. The aqueous layer was extracted with EtOAc twice. The extracts were combined and were washed with brine, dried over MgSO₄, filtered, and concentrated under reduced pressure. The residue was purified by hexane/EtOAc (4 : 1) column chromatography on silica gel to obtain (5-(azidomethyl)-2,2-dimethyl-1,3-dioxan-5-yl)methanol (6) (280 mg, 1.39 mmol, 93 %) as a colorless oil. 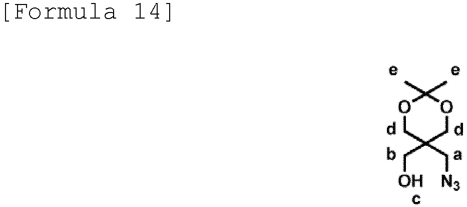 ¹H NMR (400 MHz, CDCl₃) δ 3.72, 3.67 (d, 2H, H-d, J = 11.8 Hz), 3.61 (s, 2H, H-b), 3.53 (s, 2H, H-a), 1.84 (s, 1H, H-c), 1.41 (d, 6H, H-e, J = 3.2 Hz)

### Synthesis of 2,3,5,6-tetrafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (10)

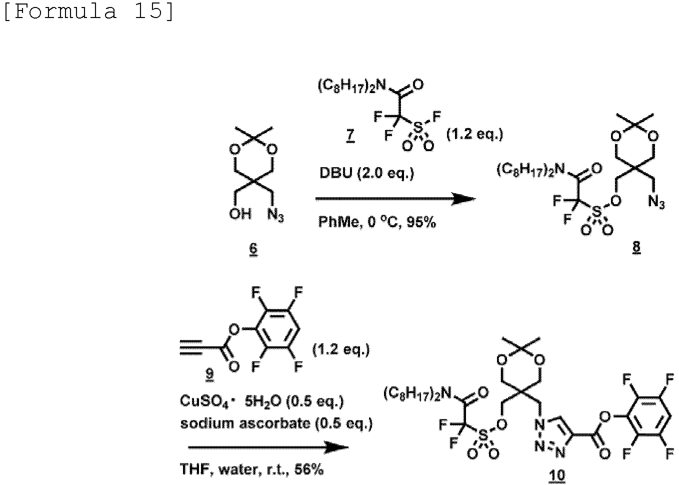

### (5-(Azidomethyl)-2,2-dimethyl-1,3-dioxan-5-yl)methyl 2-(dioctylamino)-1,1-difluoro-2-oxoethane-1-sulfonate (8)

1,8-Diazabicyclo[5.4.0]undec-7-ene (49.0 µL, 348 µmol, 1.08 g/mL, 2.0 eq.) was added to a stirring solution of (5-(azidomethyl)-2,2-dimethyl-1,3-dioxan-5-yl)methanol (6) (35.0 mg, 174 µmol, 1.0 eq.) and 2-(dioctylamino)-1,1-difluoro-2-oxoethane-1-sulfonyl fluoride (7) (110 mg, 191 µmol, 1.1 eq.) in PhMe (1 mL) at 0°C in an Ar atmosphere. The mixture was stirred at 0°C for 10 minutes, and the reaction mixture was then poured in NH₄Cl aq. and EtOAc. The aqueous layer was extracted with EtOAc twice. The extracts were combined and were washed with brine, dried over MgSO₄, filtered, and concentrated under reduced pressure. The residue was purified by chromatography using hexane/EtOAc (9 : 1) in the silica gel column to obtain (5-(azidomethyl)-2,2-dimethyl-1,3-dioxan-5-yl)methyl 2-(dioctylamino)-1,1-difluoro-2-oxoethane-1-sulfonate (8) (96.7 mg, 166 µmol, 95%) in a colorless oil form. 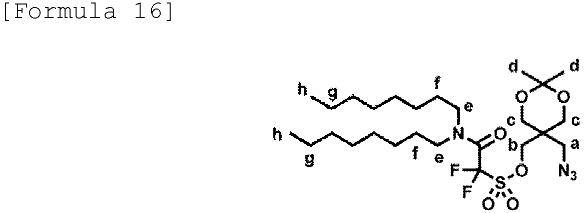 ¹H NMR (400 MHz, CDCl₃) δ 4.53 (s, 2H, H-b), 3.72 (s, 4H, H-c), 3.57-3.42 (m, 4H, H-e), 1.51-1.66 (m, 4H, H-f), 1.42 (d, 6H, H-d, J = 2.5 Hz), 1.21-1.35 (m, 20H, H-g), 0.85-0.92 (m, H-h, 6H)

### 2,3,5,6-Tetrafluorophenyl propionate (9)

DCC (410 mg, 1.99 mmol, 1.1 eq.) and DMAP (22.1 mg, 181 µmol, 0.1 eq.) in DCM (1.5 mL) were added to a stirring solution of 2,3,5,6-tetrafluorophenol (300 mg, 1.81 mmol, 1.0 eq.) and propionic acid (165 mg, 2.35 mmol, 1.3 eq.) in DCM (1.5 mL) at 0°C in an Ar atmosphere. The mixture was stirred at room temperature for 24 hours, and the reaction mixture was then filtered through a Celite layer, and the filtrate was poured in 1 M HCl aq. and EtOAc. The aqueous layer was extracted with EtOAc twice. The extracts were combined and were washed with brine, dried over MgSO₄, filtered, and concentrated under reduced pressure. The residue was purified by hexane/EtOAc (9 : 1) column chromatography on silica gel to obtain 2,3,5,6-tetrafluorophenyl propionate (9) (223 mg, 1.02 mmol, 57%) as a colorless oil. 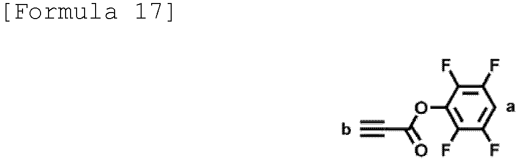 ¹H NMR (400 MHz, CDCl₃) δ 7.06 (tt, 1H, H-a, J = 9.8 Hz, 7.1 Hz), 3.24 (s, 1H, H-b)

### 2,3,5,6-Tetrafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (10)

Copper(II) sulfate pentahydrate (4.28 mg, 17.2 µmol, 0.5 eq.) and sodium L-ascorbate (3.40 mg, 17.2 µmol, 0.5 eq.) in water (100 µL) were added to a solution of (5-(azidomethyl)-2,2-dimethyl-1,3-dioxan-5-yl)methyl 2-(dioctylamino)-1,1-difluoro-2-oxoethane-1-sulfonate (8) (20.0 mg, 34.3 µmol, 1.0 eq.) and 2,3,5,6-tetrafluorophenyl propionate (9) (11. 2 mg, 51.5 µmol, 1.5 eq.) in THF (400 µL). The mixture was stirred at room temperature for 24 hours, and the reaction mixture was then poured in NaHCO₃ aq. and EtOAc. The aqueous layer was extracted with EtOAc twice. The extracts were combined and were washed with brine, dried over MgSO₄, filtered, and concentrated in vacuum. The residue was purified by hexane/EtOAc (9 : 1) column chromatography on silica gel to obtain 2,3,5,6-tetrafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (10) (15.5 mg, 19.4 µmol, 56%) as a colorless oil. 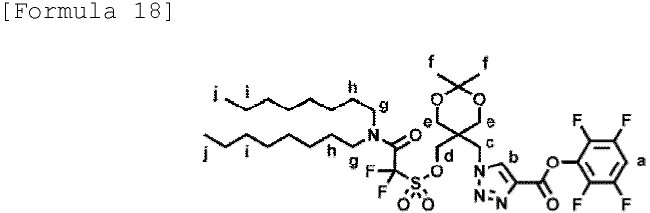 ¹H NMR (400 MHz, CDCl₃) δ 8.63 (s, 1H, H-b), 7.09 (tt, 1H, H-a, J = 10 Hz, 7.2 Hz), 4.71 (s, 2H, H-c), 4.50 (s, 2H, H-d), 3.87, 3.73 (d, 2H, H-e, J = 12.4 Hz), 3.41 (m, 4H, H-g), 1.68-1.53 (m, 4H, H-h), 1.50 (d, 6H, H-f, J = 9.2 Hz), 1.22-1,37 (m, 20H, H-i), 0.86-0.94 (m, 6H, H-j)

### Synthesis of tert-butyl (6-(1-((5-(iodomethyl)-2,2-dimethyl-1,3-dioxan-5-yl)methyl)-1H-1,2,3-triazole-4-carboxamide)-1-((4-methoxyphenyl)amino)-1-oxohexan-2-yl)carbamate (13)

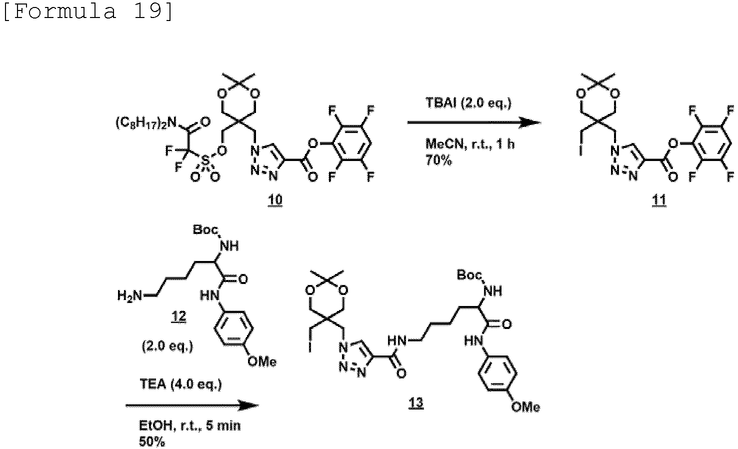

### 2,3,5,6-Tetrafluorophenyl 1-((5-(iodomethyl)-2,2-dimethyl-1,3-dioxan-5-yl)methyl)-1H-1,2,3-triazole-4-carboxylate (11)

Tetrabutylammonium iodide (120 mg, 325 µmol, 2.0 eq.) was added to a stirring solution of 2,3,5,6-tetrafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (10) in MeCN (1 mL) at room temperature. The mixture was stirred at room temperature for 1 hour, and the reaction mixture was then poured in NH₄Cl aq. and EtOAc. The aqueous layer was extracted with EtOAc twice. The extracts were combined and were washed with brine, dried over MgSO₄, filtered, and concentrated under reduced pressure. The residue was purified by hexane/EtOAc (9 : 1) column chromatography on silica gel to obtain 2,3,5,6-tetrafluorophenyl 1-((5-(iodomethyl)-2,2-dimethyl-1,3-dioxan-5-yl)methyl)-1H-1,2,3-triazole-4-carboxylate (11) (60.0 g, 113 µmol, 70%). 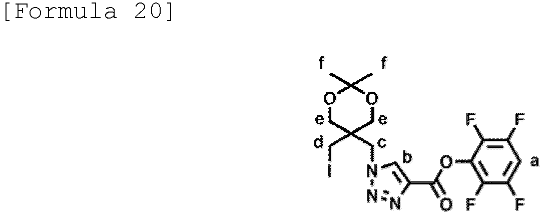 ¹H NMR (400 MHz, CDCl₃) δ 8.45 (s, 1H, H-b), 7.07 (tt, 1H, H-a, J = 10 Hz, 7.3 Hz), 4.77 (s, 2H, H-c), 3.82 (d, 2H, H-e, J = 12.4 Hz), 3.82, 3.58 (d, 2H, H-e, J = 12.4 Hz), 3.09 (s, 2H, H-d), 1.49 (d, 6H, H-f)

### tert-Butyl (6-(1-((5-(iodomethyl)-2,2-dimethyl-1,3-dioxan-5-yl)methyl)-1H-1,2,3-triazole-4-carboxamide)-1-((4-methoxyphenyl)amino)-1-oxohexan-2-yl)carbamate (13)

Triethylamine (31.6 µL, 0.726 g/mL, 227 µmol, 4 eq.) and tert-butyl (6-amino-1-((4-methoxyphenyl)amino)-1-oxohexan-2-yl)carbamate (12) (39.8 mg, 113 µmol, 2.0 eq.) were added to a stirring solution of 2,3,5,6-tetrafluorophenyl 1-((5-(iodomethyl)-2,2-dimethyl-1,3-dioxan-5-yl)methyl)-1H-1,2,3-triazole-4-carboxylate (11) (30.0 mg, 56.7 µmol, 1.0 eq.) in EtOH (100 µL) at room temperature. The mixture was stirred at room temperature for 10 minutes, and the reaction mixture was then poured in NH₄Cl aq. and EtOAc. The aqueous layer was extracted with EtOAc twice. The extracts were combined and were washed with brine, dried over MgSO₄, filtered, and concentrated under reduced pressure. The residue was purified by column chromatography using CH₃Cl/MeOH (9 : 1) on silica gel to obtain tert-butyl (6-(1-((5-(iodomethyl)-2,2-dimethyl-1,3-dioxan-5-yl)methyl)-1H-1,2,3-triazole-4-carboxamide)-1-((4-methoxyphenyl)amino)-1-oxohexan-2-yl)carbamate (13) (20.3 mg, 28.4 µmol, 50%). 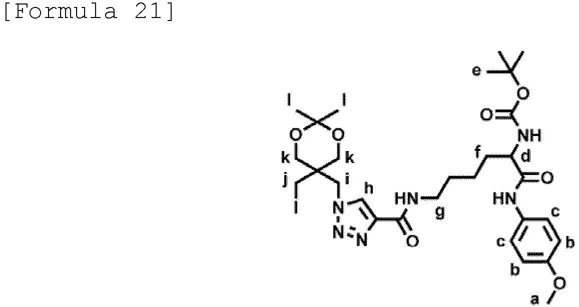 ¹H NMR (400 MHz, CD₃OD) δ 8.34 (s, 1H, H-h), 7.42 (d, 2H, H-c, J = 8.8 Hz), (d, 2H, H-c, J = 8.8 Hz), 4.68 (s, 2H, H-i), 4.08-4.12 (m, 1H, H-d), 3.81, 3.61 (d, 2H, H-k, J = 12.2), 3.42 (t, 2H, H-g, J = 6.9 Hz), 3.19 (s, 2H, H-j), 1.47-1.90 (m, 6H, H-f), 1.45 (d, 6H, H-l, J = 3.2 Hz), 1.44 (brs, 9H, H-e)

### Synthesis of 2,3,4,5,6-pentafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (14)

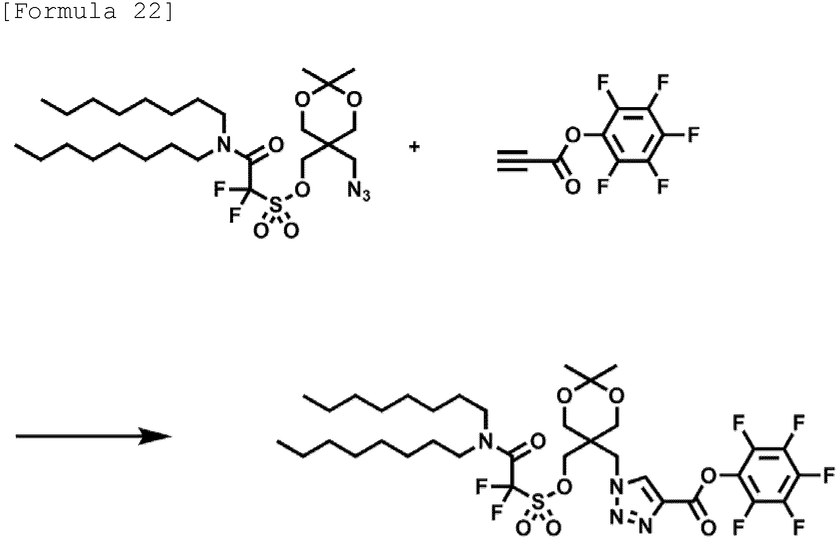

### 2,3,4,5,6-Pentafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (14)

Copper(II) sulfate pentahydrate (5.89 mg, 23.6 µmol, 0.5 eq.) and sodium L-ascorbate (4.76 mg, 17.2 µmol, 0.5 eq.) in water (100 µL) were added to a solution of (5-(azidomethyl)-2,2-dimethyl-1,3-dioxan-5-yl)methyl 2-(dioctylamino)-1,1-difluoro-2-oxoethane-1-sulfonate (27.5 mg, 47.2 µmol, 1.0 eq.) and perfluorophenyl propionate (16. 7 mg, 70.8 µmol, 1.5 eq.) in THF (400 µL). The mixture was stirred at room temperature for 24 hours, and the reaction mixture was then poured in NaHCO₃ aq. and EtOAc. The aqueous layer was extracted with EtOAc twice. The extracts were combined and were washed with brine, dried over MgSO₄, filtered, and concentrated in vacuum. The residue was purified by hexane/EtOAc (9 : 1) column chromatography on silica gel to obtain 2,3,4,5,6-pentafluorophenyl 1-((5-((((2-(dioctylamino)-1,1-difluoro-2-oxoethyl)sulfonyl)oxy)methyl)-2,2-dimethyl-1,3-dioxan-5-yl)methyl)-1H-1,2,3-triazole-4-carboxylate (14) (10.4 mg, 12.7 µmol, 27%) as a colorless oil.

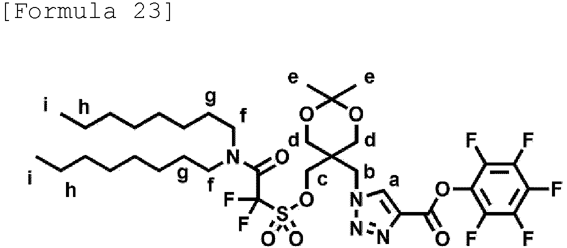

### [Example 2] Synthesis of radioactive halogen-labeled compound

### Production of compound (3) [¹²⁵I] 2,3,5,6-tetrafluorophenyl 1-(3-hydroxy-2-(hydroxymethyl)-2-(iodomethyl)propyl)-1H-1,2,3-triazole-4-carboxylate

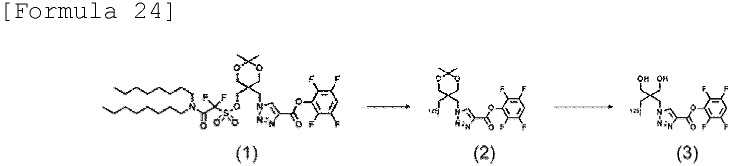

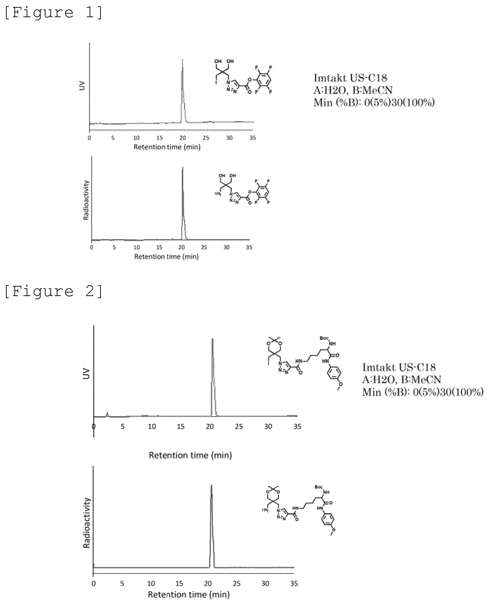

The compound (1) (0.4 mg, 5.0×10⁻⁷ mol) was dissolved in a 1% DIEA/MeCN solution (50 µL). A [¹²⁵I]NaI aqueous solution (0.5 µL, 61.6 µCi) was added to the resulting solution, followed by reaction at 50°C for 1 hour to produce compound (2). Continuously, the solution containing the compound (2) was applied to C18-ODS SEP-PAK (50 mg) manufactured by Watars Corporation and washed with water (1000 µL). Subsequently, 6 M hydrochloric acid (2000 µL) was poured into the column, and the column filled with 6 M hydrochloric acid was left to stand at room temperature for 10 minutes. After washing with water (1000 µL), extraction with MeCN (150 µL) was performed to obtain compound (3) with a radiochemical yield of 76.5% (two-step). It was confirmed that the radiochemical purity was higher than 99% by an analysis system of HPLC using Imtakt Unison US-C18 150 × 4.6 mm and a mobile phase composed of MilliQ as the A-phase and MeCN as the B-phase and changing from A-phase: 95% and B-phase: 5% to A-phase: 0% and B-phase: 100% for a period of from 0 to 30 minutes (Figure 1).

### Production of compound (4) [¹²⁵I] tert-butyl (6-(1-((5-(iodomethyl)-2,2-dimethyl-1,3-dioxan-5-yl)methyl)-1H-1,2,3-triazole-4-carboxamide)-1-((4-methoxyphenyl)amino)-1-oxohexan-2-yl)carbamate

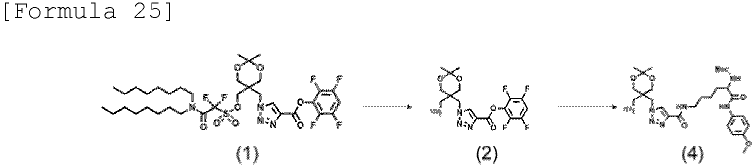

The compound (1) (0.4 mg, 5.0×10⁻⁷ mol) was dissolved in a 1% DIEA/MeCN solution (50 µL). A [¹²⁵I]NaI aqueous solution (0.5 µL, 59 µCi) was added to the resulting solution, followed by reaction at 50°C for 1.5 hours. The solution was applied to Oasis HLB 1 cc Vac Cartridge (10 mg) manufactured by Waters Corporation and washed with water (500 µL). Subsequently, MeCN was poured into the cartridge, and compound (2) was collected in a 30 to 60 µL fraction. A tert-butyl (6-amino-1-((4-methoxyphenyl)amino)-1-oxohexan-2-yl)carbamate ethanol solution (2.0 mg/mL, 30 µL) was added to this solution, followed by reaction at 37°C for 1 hour to obtain compound (4) with a radiochemical yield of 64.8% (two-step). It was confirmed that the radiochemical purity was higher than 99% by an analysis system of HPLC using Imtakt Unison US-C18 150 × 4.6 mm and a mobile phase composed of MilliQ as the A-phase and MeCN as the B-phase and changing from A-phase: 95% and B-phase: 5% to A-phase: 0% and B-phase: 100% for a period of from 0 to 30 minutes (Figure 2).

### [Example 3] Yield of radioactive iodine compound having triazole group

The yield of a radioactive iodine compound having a triazole group (compound A) was compared to those of other compounds (compounds B and C).

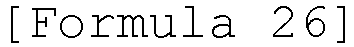

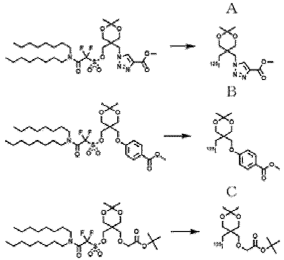

A precursor (0.3 mg, 5.0×10⁻⁷ mol) of each compound was dissolved in a 1% DIEA/MeCN solution (50 µL). A [¹²⁵I]NaI aqueous solution (0.5 µL, 3 µCi) was added to this solution, followed by reaction at 50°C for 40 minutes. The reaction solution was analyzed by normalphase TLC (toluene : ethyl acetate = 4 : 1 (compounds B and C) or toluene : ethyl acetate = 7 : 3 (compound A)).

The yields of the compounds B and C were 49.2% and 23.8%, respectively. In contrast, the yield of the compound A having a triazole group was 84.4%, which was significantly high compared to those of the compound B and C.

### [Example 4] Production of iodine-labeled antibody Synthesis of ¹²⁵I iodine-labeled compound

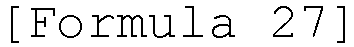

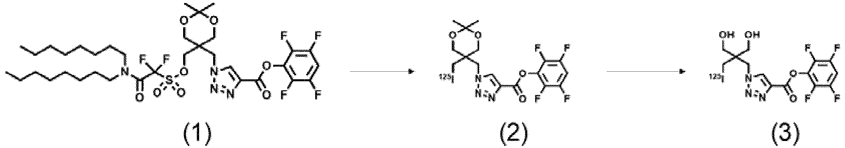

The compound (1) (0.4 mg) was dissolved in a 1% DIEA/MeCN (50 µL). Furthermore, [¹²⁵I]NaI aq. (0.5 µL, 36.8 µCi) was added thereto, followed by reaction at 50°C for 3 hours. The whole amount of the reaction solution was added to C18-ODS SEP-PAK (50 mg) manufactured by Watars Corporation activated with methanol. Subsequently, the SEP-PAK was washed with water (1000 µL). Furthermore, 6 M hydrochloric acid (2000 µL) was added to the SEP-PAK, and the column filled with 6 M hydrochloric acid was left to stand at room temperature for 10 minutes. Elution from the column was performed using MeCN : H₂O = 1 : 1 (500 µL) to obtain compound (3) (26.0 µCi) (radiochemical yield: 70%).

### Production of labeled antibody

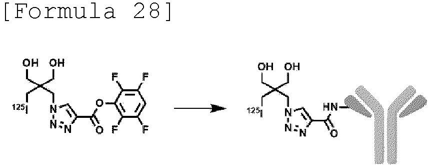

The solution of the compound (3) was subjected to distillation with nitrogen gas. An EGFR antibody solution (2 mg/mL, 200 µL, 0.16 M borate buffer solution, pH 8 to 9) was added to the container. The advance of the reaction was confirmed by TLC. After 60 minutes, purification using a spin column (Sephadex G50, 0.1 M phosphoric acid, pH 7.4) was performed at 1500 rpm for 2 min to obtain a radioactive-labeled antibody (8.85 µCi) (radiochemical yield: 33%).

### [Example 5] Synthesis of ²¹¹At astatine labeled compound

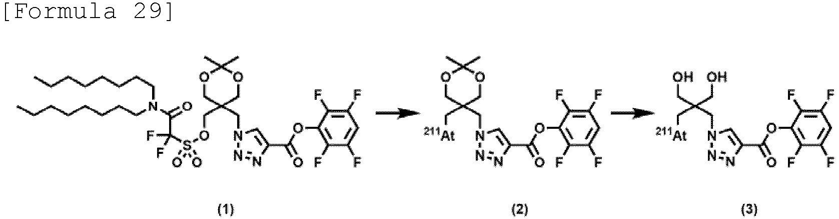

The compound (1) (0.4 mg) was dissolved in a 1% DIEA/MeCN (50 µL) and a 10% sodium ascorbate aq. (0.5 µL). Furthermore, [²¹¹At]At⁻ in MeCN (5 µL, 0.455 MBq) was added thereto, followed by reaction at 50°C for 1 hour. The whole amount of the reaction solution was added to C18-ODS SEP-PAK (50 mg) manufactured by Watars Corporation activated with methanol. Subsequently, the SEP-PAK was washed with water (1000 µL). Furthermore, 6 M hydrochloric acid (2000 µL) was added to the SEP-PAK, and the column filled with 6 M hydrochloric acid was left to stand at room temperature for 10 minutes. Elution from the column was performed using MeCN : H₂O = 1 : 1 (500 µL) to obtain compound (3) having 0.244 MBq (radiochemical yield: 53%).

All publications, patents, and patent applications cited herein are hereby incorporated by reference in their entirety.

### [Industrial Applicability]

Since radioactive halogen-labeled compounds can be used as medicines, the present invention can be used in a medicine-related field.

## Claims

1. A compound represented by a following general formula (I): 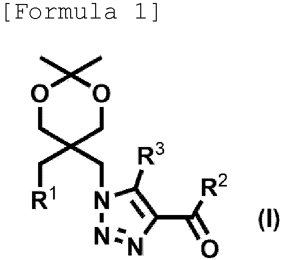 wherein, R¹ represents a leaving group capable of nucleophilic substitution by a halide ion, R² represents a leaving group such that nucleophilic acyl substitution reaction is advanced by an amino group, and R³ represents a hydrogen atom or a halogen atom.

2. The compound according to Claim 1, wherein R¹ in the general formula (I) is a group represented by a following general formula (A) or (B): 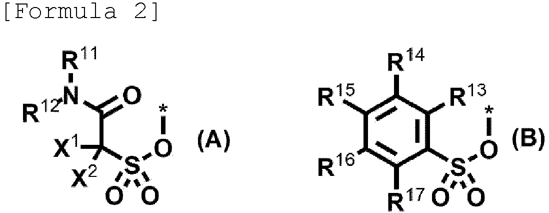 wherein, R¹¹ and R¹² each independently represent an alkyl group having 5 to 20 carbon atoms, X¹ and X² each independently represent a halogen atom, R¹⁵ represents an alkyl group having 4 to 24 carbon atoms or -CONR¹⁸R¹⁹ [wherein, R¹⁸ and R¹⁹ each independently represent an alkyl group having 1 to 24 carbon atoms or an aryl group optionally substituted with a substituent], R¹³, R¹⁴, R¹⁶, R¹⁷, and R¹⁸ each independently represent a hydrogen atom, an alkyl group having 1 to 4 carbon atoms, or an alkoxy group having 1 to 4 carbon atoms, and * represents a binding site.

3. The compound according to Claim 1, wherein R² in the general formula (I) is a group represented by a following general formula (C), (D), (E), or (F): 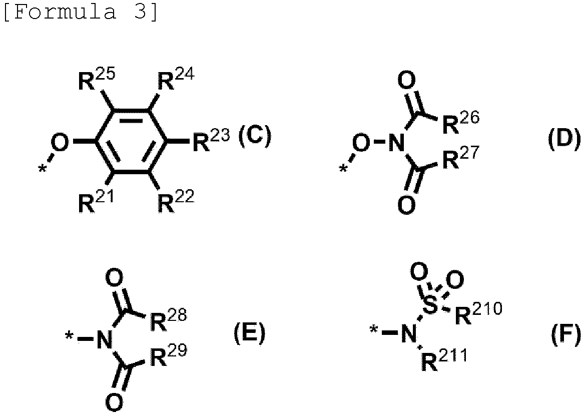 wherein, R²¹, R²², R²³, R²⁴, and R²⁵ each independently represent a hydrogen atom or a halogen atom, R²⁶ and R²⁷ each independently represent a hydrogen atom, an alkyl group having 1 to 4 carbon atoms, or an aryl group optionally substituted with a substituent [R²⁶ and R²⁷ optionally bind to each other to form a ring], R²⁸ and R²⁹ each independently represent a hydrogen atom, an alkyl group having 1 to 4 carbon atoms, an alkoxy group having 1 to 4 carbon atoms, an amino group [one or two hydrogen atoms of the amino group are optionally replaced with an alkyl group having 1 to 4 carbon atoms], or an aryl group optionally substituted with a substituent [R²⁸ and R²⁹ optionally bind to each other to form a ring], R²¹⁰ and R²¹¹ each independently represent a hydrogen atom, an alkyl group having 1 to 4 carbon atoms, or an aryl group optionally substituted with a substituent [R²¹⁰ and R²¹¹ optionally bind to each other to form a ring], and * represents a binding site.

4. A compound represented by a following general formula (IIa) or (IIb): 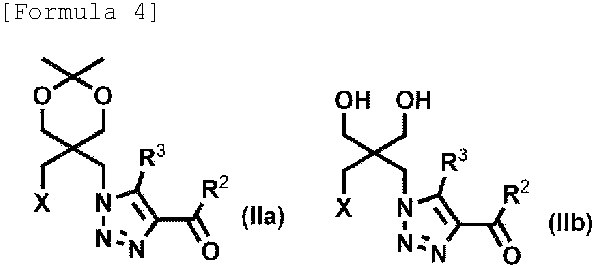 wherein, X represents a radioactive halogen atom, R² represents a leaving group such that nucleophilic acyl substitution reaction is advanced by an amino group, and R³ represents a hydrogen atom or a halogen atom.

5. The compound according to Claim 4, wherein X in the general formula (IIa) or (IIb) is a radioactive bromine atom, a radioactive iodine atom, or a radioactive astatine atom.

6. The compound according to Claim 4, wherein R² in the general formula (IIa) or (IIb) is a group represented by a following general formula (C), (D), (E), or (F): 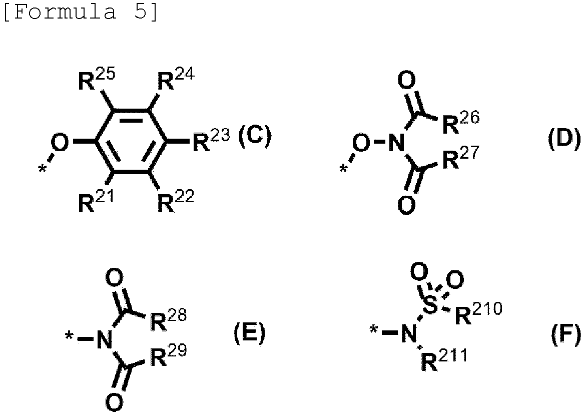 wherein, R²¹, R²², R²³, R²⁴, and R²⁵ each independently represent a hydrogen atom or a halogen atom, R²⁶ and R²⁷ each independently represent a hydrogen atom, an alkyl group having 1 to 4 carbon atoms, or an aryl group optionally substituted with a substituent [R²⁶ and R²⁷ optionally bind to each other to form a ring], R²⁸ and R²⁹ each independently represent a hydrogen atom, an alkyl group having 1 to 4 carbon atoms, an alkoxy group having 1 to 4 carbon atoms, an amino group [one or two hydrogen atoms of the amino group are optionally replaced with an alkyl group having 1 to 4 carbon atoms], or an aryl group optionally substituted with a substituent [R²⁸ and R²⁹ optionally bind to each other to form a ring], R²¹⁰ and R²¹¹ each independently represent a hydrogen atom, an alkyl group having 1 to 4 carbon atoms, or an aryl group optionally substituted with a substituent [R²¹⁰ and R²¹¹ optionally bind to each other to form a ring], and * represents a binding site.

7. A compound represented by a following general formula (IIIa) or (IIIb): 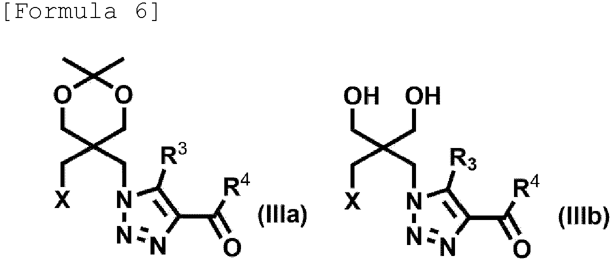 wherein, X represents a radioactive halogen atom, R³ represents a hydrogen atom or a halogen atom, R⁴ represents a monovalent group derived from a physiologically active substance.

8. The compound according to Claim 7, wherein X in the general formula (IIIa) or (IIIb) is a radioactive bromine atom, a radioactive iodine atom, or a radioactive astatine atom.

9. The compound according to Claim 7, wherein R⁴ in the general formula (IIIa) or (IIIb) is a monovalent group derived from a physiologically active substance that accumulates at a disease site.

10. The compound according to Claim 7, wherein R⁴ in the general formula (IIIa) or (IIIb) is a monovalent group derived from an antibody.

11. A method for manufacturing the compound according to any one of Claims 4 to 6, comprising a step of reacting the compound according to any one of Claims 1 to 3 with a radioactive halide ion.

12. A method for manufacturing the compound according to any one of Claims 7 to 10, comprising a step of reacting the compound according to any one of Claims 4 to 6 with a physiologically active substance.
